(19) Europäisches Patentamt
European Patent Office
Office européen des brevets

(11) Numéro de publication : **0 453 473 B1**

(12) # FASCICULE DE BREVET EUROPEEN

(45) Date de publication du fascicule du brevet : **15.09.93 Bulletin 93/37**

(21) Numéro de dépôt : **90901826.9**

(22) Date de dépôt : **09.01.90**

(86) Numéro de dépôt international : **PCT/FR90/00015**

(87) Numéro de publication internationale : **WO 90/08172 26.07.90 Gazette 90/17**

(51) Int. Cl.$^5$ : **C08J 7/12,** B01J 20/26, B01J 20/30, B01D 15/08, // G01N33/545, C12Q1/68, G01N33/80

(54) **SUPPORT D'IMMUNO-EPURATION SELECTIVE ET PROCEDE DE TRAITEMENT DE CE SUPPORT.**

(30) Priorité : **10.01.89 FR 8900415**

(43) Date de publication de la demande : **30.10.91 Bulletin 91/44**

(45) Mention de la délivrance du brevet : **15.09.93 Bulletin 93/37**

(84) Etats contractants désignés : **AT BE CH DE DK ES FR GB IT LI LU NL SE**

(56) Documents cités :
**EP-A- 0 085 016**
**EP-A- 0 141 771**
**EP-A- 0 145 386**
**EP-A- 0 173 500**
**EP-A- 0 247 975**
**FR-A- 2 321 520**
**L F Fieser et al: 'Advanced Organic Chemistry', 1961, Reinhold Publishing Corp (New York, US), see p 501-502-Curtius Recation**

(56) Documents cités :
**Chemical Abstracts, vol 98, nr 2, 10 January 1983 (Columbus, Ohio, US) T Ouchi et al: 'Vinyl polymerization. 411. Experimental supports for the concept of hard and soft hydrophobic areas and monomers in the so-called uncatalysed polymerization', see p 4, abstract nr 4843y**
**Chemical Abstracts, vol 91, nr 3, 16 July 1979 (Columbus, Ohio, US) U Seitz et al: 'New reactive microgels as carriers of enzymes', see page 260, abstract nr 15911a**

(73) Titulaire : **TRAEGER, Jules**
**A.U.R.A.L., 8, impasse Lindberg**
**F-69008 Lyon (FR)**

(72) Inventeur : **TRAEGER, Jules**
**A.U.R.A.L., 8, impasse Lindberg**
**F-69008 Lyon (FR)**
Inventeur : **SERRES, Pierre François**
**INDICIA, 87, rue de la République**
**F-69600 Oullins (FR)**

(74) Mandataire : **Ropital-Bonvarlet, Claude**
**Cabinet BEAU DE LOMENIE, 51, avenue Jean-Jaurès**
**F-69007 Lyon (FR)**

EP 0 453 473 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne le domaine technique de l'épuration de substances indésirables présentes dans un liquide par mise en oeuvre des réactions spécifiques antigène-anticorps. Plus précisément la présente invention concerne un support d'immuno-épuration sélective ainsi qu'un procédé de traitement de ce support.

La technique d'immuno-épuration sélective met en oeuvre le principe de reconnaissance spécifique d'un anticorps par un antigène et inversement.

Les anticorps ou antigènes peuvent être d'origine naturelle ou synthétique.

Les anticorps d'origine naturelle sont obtenus par introduction dans un organisme humain ou animal d'un élément étranger ou antigène qui provoque en retour la fabrication par cet organisme d'une substance spécifique appelée anticorps avec laquelle l'antigène peut s'unir électivement. D'autre part, on sait fabriquer des anticorps très spécifiques contre un antigène par voie monoclonale, et on sait fabriquer des antigènes synthétiques reproduisant fidèlement des épitopes qui sont des sites de reconnaissance de l'anticorps situés sur l'antigène.

Cette réaction spécifique entre un antigène (naturel ou synthétique) et un anticorps (naturel ou synthétique) est maintenant utilisée dans de nombreux domaines.

Ainsi, par exemple, dans le domaine du diagnostic, de nombreux tests qualitatifs ou quantitatifs sont basés sur la spécificité de ces réactions immunologiques.

Cette reconnaissance spécifique entre un antigène et un anticorps peut également être utilisée dans les techniques d'épuration de liquide.

En effet, de nombreuses pathologies sont associées à la présence dans le sang de substances indésirables. La solution proposée, il y a quelques années pour éliminer ces substances indésirables était un remplacement du sang total, ce qui nécessitait outre de grandes quantités de sang, des procédés de mise en oeuvre difficile et risquée.

Cette pratique a maintenant été remplacée par la plasmaphérèse qui est une technique de séparation des éléments cellulaires du plasma sanguin. Le plasma contenant les substances indésirables du sang est élimine, alors que les cellules sont réinjectées au patient.

Le plasma est remplace dans l'organisme par une injection de solution aqueuse d'albumine.

Cette technique de plasmaphérèse, bien que représentant un très grand progrès par rapport à la technique de remplacement du sang total présente cependant de nombreux inconvénients, parmi lesquels on peut citer :

- un coût élevé,
- des réactions de "choc" fréquentes dues à l'injection de liquides de substitution, provenant du manque de biocompatibilité des solutions de substitution,
- des risques de transmission d'hépatite ou de SIDA par les concentrés d'albumine,
- une privation du patient des potentiels d'une thérapie médicamenteuse simultanée car l'ensemble du plasma est soustrait au patient,
- un disfonctionnement plaquettaire .

Mais le principal inconvénient de la plasmaphérèse est son manque de sélectivité vis à vis des substances que l'on souhaite éliminer, ce qui conduit dans certains cas à retirer au patient des composants utiles dans le traitement même de la maladie. Ainsi, par exemple, dans le cadre du traitement du Lupus Erythémateux Disséminé, l'épuration sélective des anticorps anti-ADN natifs s'impose par rapport à la plasmaphérèse. En effet, chez le malade lupique, il y a un dérèglement de la balance entre les anticorps anti-ADN et les anticorps anti-idiotypes anti-ADN. En d'autres termes, la concentration très élevée d'immunoglobulines anti-ADN natifs ne peut plus être controlée par le système de régulation anti-idiotypique anti ADN qui est largement déborde. Il est donc indispensable d'éliminer sélectivement les immunoglobulines G anti-ADN et de conserver les anti-idiotypes. Or cette sélection n'est pas possible à réaliser avec la technique de plasmaphérèse seule.

L'efficacité de la technique d'épuration est ainsi largement dépendante de la sélectivité possible des substances à éliminer.

D'autre part, il est souhaitable dans le cas où la technique d'épuration est utilisée pour des liquides biologiques, tels que du sang ou du plasma sanguin, de s'assurer que le traitement effectue n'entraine aucune détérioration des autres substances présentes dans le liquide.

Les résultats obtenus grâce à cette technique d'épuration dépendent donc à la fois des molécules épuratrices choisies, du support sur lequel seront fixées ces molécules, ainsi que de la qualité de fixation des molécules épuratrices sur le support.

Le document EP-A-141 711 décrit notamment des conjugués élaborés par fixation d'un ligand sur un support insoluble qui est un support particulaire greffe en surface, c'est-à-dire obtenu par greffage sur des particules d'un polymère insoluble de molécules insaturées capables de se polymériser et portant une fonction organique substituable du type allylamine, acrylamide ou acide acrylique.

Le document EP-A-247 975 décrit un support polymérique pour réaction topochimique à partir d'un homo ou d'un copolymère d'acrylonitrile modifié par l'introduction de nouveaux groupes réactifs autres que l'acrylozoture.

Les solutions proposées jusqu'ici dans l'art antérieur présentent de nombreux inconvénients et ne donnent pas entièrement satisfaction.

L'objet de la présente invention est donc de proposer un support ainsi qu'un procédé de traitement de ce support pour l'épuration sélective de substances indésirables présentes dans les liquides biologiques de l'organisme.

Un autre objet de la présente invention est de proposer un support ainsi qu'un procédé de traitement de ce support permettant l'extraction spécifique des substances d'origine biologique de tout liquide dans des buts de biochimie analytique ou préparative.

Un autre objet de la présente invention est de proposer un support ainsi qu'un procédé de traitement de ce support pour l'immuno-épuration sélective d'un liquide présentant une bonne compatibilité avec le liquide à traiter.

Un autre objet de la présente invention est de proposer un support ainsi qu'un procédé de traitement de ce support d'épuration permettant une bonne mouillabilité par le liquide à traiter.

Un autre objet de la présente invention est de proposer un support ainsi qu'un procédé de traitement de ce support permettant de réaliser le traitement du liquide avec un bon rendement.

D'autres objets et avantages de la présente invention apparaitront à la lecture de la description qui va suivre.

Afin de réaliser ces objets, la présente invention propose un support pour l'immunoépuration sélective d'un liquide constitué par un copolymère d'au moins un monomère comprenant au moins une fonction choisie parmi les fonctions acide, amide, hydrazide ou ester, dans laquelle la fonction carbonyle C = 0 est reliée à la chaine polymérique par R pouvant être un alcoyle, un aryle ou un simple lien valenciel et d'un monomère à au moins un groupement apportant de l'hydrophobie, caractérisé en ce que ladite fonction acide amide hydrazide ou ester est transformée en acyl-azoture..

La présente invention a également pour objet un procédé de traitement d'un support pour l'immunoépuration sélective d'un liquide, ledit support étant constitué par un copolymère d'au moins un monomère comprenant au moins une fonction choisie parmi les fonctions acide, amide, hydrazide ou ester, dans lesquelles la fonction carbonyle C = 0 est reliée à la chaine polymérique par R pouvant être un alcoyle, un aryle ou un simple lien valenciel et d'un monomère à au moins un groupement apportant de l'hydrophobie, caractérisé en ce qu'il consiste à transformer l'une au moins desdites fonctions en groupements acyl-azotures.

Selon un mode particulier du procédé de l'invention, l'une au moins desdites fonctions acide, amide ou ester est transformée en hydrazide grâce à l'action de l'hydrazine, et ladite hydrazide est transformée en acrylazoture grâce à l'action du nitrite de sodium en milieu acide.

## Nature du support utilisé

La nature du support utilise dans la présente invention doit permettre, sinon la fixation immédiate des molécules épuratrices choisies, du moins l'activation chimique des groupements fonctionnels présents sur le support afin qu'ils puissent ensuite réagir, lors de la phase de greffage, avec certains groupements chimiques présents sur les molécules épuratrices.

Selon la présente invention, on choisit d'utiliser à cette fin des polymères dont au moins un monomère possède au moins un groupement fonctionnel tel qu'un groupement acide, amide, hydrazide ou ester, dans lequel la fonction carbonyle C = 0 est reliée à la chaîne polymérique par R qui peut être un groupement alcoylique, arylique ou un simple lien valenciel. Ces groupements fonctionnels pourront être activés chimiquement selon une méthode qui sera décrite ci-après.

Avantageusement, le support utilisé comprend entre 0,3 et 3 équivalents de groupement acide, amide, hydrazide ou ester par kg de polymère.

Afin de permettre de traiter des liquides biologiques provenant de l'organisme grâce au support selon la présente invention, il est particulièrement avantageux que ce support présente d'excellentes qualités de biocompatibilité.

Le support utilise dans la présente invention présente en outre avantageusement un caractère hydrophobe qui favorise dans la plupart des cas l'orientation favorable des molécules épuratrices à fixer. En effet, lorsque l'on fixe la molécule épuratrice, il est important de conserver disponible son site de reconnaissance et de fixation de la molécule à épurer. Ainsi, dans le cas où la molécule épuratrice est une immunoglobuline G présentant un fragment hydrophobe, le fragment Fc, et deux fragments Fab de reconnaissance de la molécule à éliminer, il est avantageux que le support favorise la fixation de l'immunoglobuline G par son fragment Fc. Cette orientation favorable de la molécule épuratrice peut être obtenue grâce à un support polymérique de balance hydrophobe/hydrophile favorable, grâce notamment à la présence de groupements apportant de l'hydrophobie au support tels que les groupements nitriles des copolymères d'acrylonitrile. Ainsi, l'orientation favorable des molécules épuratrices fixées permettra, au moment de l'épuration, d'obtenir des rendements proches de 100% entre la quantité de molécules épuratrices fixées et la quantité de molécules épurées, chaque molécule épuratrice étant capable de reconnaitre et de se lier avec une molécules spécifique à épurer.

Si le caractère hydrophobe du support est important pour permettre une orientation favorable de la molécule épuratrice fixée, ce support doit cependant présenter en outre une certaine hydrophilie permettant de le "mouiller" facilement, i.e. de l'imprégner par le liquide à traiter.

L'orientation favorable des immunoglobulines fixées permet d'accroitre la biocompatibilité du support. En effet, un support présentant une hydrophilie importante, tel que celui décrit dans le brevet US-A-4 693 985 favoriserait une fixation de l'immunoglobuline G qui laisserait le fragment Fc libre. Ce fragment Fc est un facteur d'activation du complément ; sa possibilité d'action présente donc des risques dans le cas où le liquide traité est un liquide biologique provenant d'un patient et destine à être retourne au patient après traitement.

Parmi les supports d'épuration convenant bien pour la présente invention, on peut citer les copolymères d'acrylonitrile et d'acrylate de méthyle ou de méthacrylate de methyle ou d'acide acrylique ou d'acrylamide ou d'acide crotonique.

Afin de permettre la fixation des molécules épuratrices en quantité suffisante pour un traitement efficace du liquide à épurer, le support utilisé doit présenter une surface disponible importante. A ce titre, on préfère utiliser dans la présente invention un support microporeux présentant une surface utile importante par rapport à la surface apparente. On peut ainsi multiplier plusieurs dizaines de fois la surface apparente.

Ainsi, il est possible de fixer des molécules épuratrices non plus uniquement à la surface du support, mais également à l'intérieur des micropores, ce qui augmente considérablement la surface active du support. L'épuration du liquide s'effectuera par contact entre les molécules épuratrices fixées et les substances à éliminer lors du passage du liquide à l'intérieur des micropores. La porosité du support est avantageusement comprise entre 500 et 10000 Angströms.

En effet, il est avantageux que la taille des pores ne soit pas trop grande afin de permettre un contact optimum entre les molécules épuratrices et les molécules à épurer. D'autre part, la taille des pores ne doit pas être trop petite pour ne pas entrainer de perte de charge trop importante lors de l'utilisation du support pour l'épuration d'un liquide.

Parmi les structures microporeuses convenant bien comme support d'immunoépuration, on peut citer la membrane fabriquée par RHONE-POULENC, désignée par la dénomination TPC 19 constituée par un copolymère acrylonitrile-méthacrylate de methyle-méthallyl sulfonate de sodium. La porosité de cette membrane est comprise entre 2000 et 5000 Angströms. La méthode de fabrication de cette membrane est décrite dans la demande de brevet EP-A-85 016 correspondant au brevet US-A-4 687 580.

La structure microporeuse utilisée avantageusement comme support pour la présente invention, peut être réalisée sous forme de membrane de type plan, dont l'épaisseur est par exemple, comprise entre 100 et 200 micromètres, ainsi que sous forme de fibres creuses.

## Activation du support

Le support d'immunoépuration utilisé dans la présente invention doit être activé chimiquement afin de permettre ensuite le greffage par liaison covalente de molécules épuratrices. En effet, pour la fixation de molécules épuratrices dont la taille est suffisante pour éviter la nécessite d'un intermédiaire chimique, le greffage par liaison covalente est la solution préférentielle car cette liaison étant plus solide que les liaisons hydrophobes ou ioniques, le risque d'entrainement des molécules épuratrices par le liquide à épurer est moins grand.

Les procédés d'activation choisis varient selon les groupements fonctionnels présents sur le support, ainsi que, selon les groupements fonctionnels de la molécule biologique épuratrice que l'on veut fixer. L'activation du support consiste par exemple à transformer l'une au moins des fonctions acide, amide ou ester du support en hydrazide grâce à l'action de l'hydrazine.

Dans le cas où le support utilisé possède des groupements acides carboxyliques, ces groupements peuvent être actives pour conduire aux azotures qui réagiront ensuite avec les molécules épuratrices. Les groupements acides carboxyliques sont tout d'abord transformés en ester ; ils sont méthylés pour conduire à des groupements carboxyméthylés. Cette phase de méthylation peut être effectuée dans un bain de méthanol acidifié durant 1 à 10 jours, de préférence 2 à 6 jours. Les meilleurs résultats ont été obtenus pour des durées de 4 jours et plus.

Dans le cas où le support utilisé possède déjà des groupements carboxyméthylés, ainsi que dans le cas où ces groupements proviennent de la méthylation des radicaux acides carboxyliques, ces groupements carboxyméthylés sont transformés en hydrazides par hydrazinolyse.
L'hydrazine peut être utilisée en solution dans de l'eau ou dans du méthanol. La concentration peut varier de 0,1 à 10% et plus particulièrement de 0,8 à 2,5%.
Ainsi que cela apparait sur le tableau I, la quantité de molécules épuratrices fixées atteint sensiblement un plateau avec une concentration en solution aqueuse d'environ 1% . Ce tableau relate la quantité d'Ig G humaines fournies par la société MERIEUX greffées en microgrammes/cm2 de surface apparente en fonction de la concentration en hydrazine de la solution aqueuse.
L'hydrazinolyse est effectuée pendant une durée de deux heures.

TABLEAU I

| CONCENTRATION EN HYDRAZINE | QUANTITE D'IgG GREFFEES EN MICROGRAMMES/CM2 |
|---|---|
| 0,2% | 3 |
| 0,5% | 8 |
| 1% | 14 |
| 2% | 13 |
| 5% | 16 |

Il serait également possible d'utiliser de l'hydrazine en solution dans du méthanol. Or, les essais effectués ont montré que, pour une mème concentration en hydrazine, les quantitées de molécules fixées sont plus importantes avec de l'hydrazine en solution aqueuse plutôt qu'en solution dans du méthanol. Etant donné que l'hydrazine est un composé toxique, il n'est pas souhaitable d'augmenter inconsidérement la quantité fournie au mélange réactionnel.
On choisit donc d'effectuer l'hydrazinolyse du support d'immunoépuration en utilisant une solution aqueuse d'hydrazine à 1 ou 2%.

L'influence de la durée de la réaction d'hydrazinolyse est illustrée sur le tableau II. On remarque que la quantité de molécules épuratrices fixées atteint un plateau après 1 heure de réaction environ.
Ce tableau relate la quantité d'Ig G humaines fournies par la société MERIEUX greffées en microgrammes/cm2 de surface apparente en fonction du temps. L'hydrazinolyse est effectuée grâce à une solution aqueuse d'hydrazine à 1%.

TABLEAU II

| TEMPS EN MN | QUANTITES D'IgG FIXES EN MICROGRAMMES/CM2 |
|---|---|
| 5 | 3 |
| 10 | 6 |
| 20 | 8 |
| 30 | 10 |
| 60 | 12 |
| 120 | 10 |
| 600 | 13 |
| 1440 | 10 |

La réaction d'hydrazinolyse est effectuée dans un bain de solution d'hydrazine, sous agitation lorsque le support utilisé se présente sous forme de film plan, ou par passage continu à travers la structure du support dans le cas où ce support possède des micropores.

La phase suivante d'activation du support consiste à transformer les groupements hydrazides $CONHNH_2$ en groupements acyl-azotures $CON_3$ très réactifs. Cette transformation est effectuée par traitement du support avec un mélange de nitrite de sodium en milieu acide chlorhydrique.
La préparation du mélange se fait de façon extemporanée en mélangeant volume à volume une solution d'acide chlorhydrique 0,3 N et une solution formée par 3,45 g de nitrite de sodium dans 100 ml d'eau stérile.
La réaction peut être effectuée à froid, c'est-à-dire entre 0°C et 4°C.
La durée choisie de la réaction est par exemple de 3 minutes lorsque le support utilisé se présente sous forme de film plan et par exemple de 5 à 15 minutes lorsqu'il s'agit d'une structure microporeuse.
On peut noter ici que la nature du support choisie est déterminante pour éviter une désintégration totale ou partielle du support lors des différents traitements chimiques. En effet, dans les mêmes conditions d'activation, des matériaux tels que le polystyrène carboxylé et le nylon se désagrègent.

Fixation de la molécule épuratrice

La réaction de fixation de la molécule épuratrice dépend à la fois des groupements chimiques activés présents sur le support ainsi que des groupements réactifs présents sur la molécule épuratrice.

Lorsque, ainsi que cela a été décrit précédemment, l'activation du support conduit à la formation de groupements acylazotures, il est possible de greffer les molécules épuratrices par réaction avec ces groupements acylazotures et notamment en liant de façon covalente une molécule épuratrice possédant un groupement amine, selon la réaction suivante :

$$- CON_3 + NH_2 - \text{molécule épuratrice}$$

- C-NH-
||
O

molécule épuratrice

Les molécules épuratrices greffées sont choisies pour leur aptitude à reconnaître et à se lier avec les substances à éliminer. Il s'agit par exemple de protéines telles que des Immunoglobulines G de lapins ou de moutons, de fragments d'ADN bicatenaires pourvus d'une extrémité monocaténaire aminée, de polysaccharides à fonction aminée, de dérivés de bas poids moléculaire d'origine biologique ou synthétique, de protéine A, de glycoprotéines, des fragments d'acide ribonucléique, etc...

Le greffage est effectue par mise en contact du support pour l'immuno-épuration sélective avec une solution de greffage constituée par des molécules épuratrices en solution. Par exemple, la réaction de fixation est effectuée par passage à travers le support d'une solution tamponnée de molécules épuratrices, pendant une durée permettant la saturation du maximum de sites actives. Un passage en continu pendant deux heures à 4°C est satisfaisant à cette fin ; une durée d'une heure devrait mème être largement suffisante car il semble que la durée de vie des sites azotures soit brève. Les molécules épuratrices sont avantageusement en solution dans du tampon dont le pH est compris entre 8 et 11, par exemple dans du tampon borate à un pH 9 ou 10, constituant ainsi ce que l'on appelle le bain de greffage. Le tampon borate est par exemple obtenu en mélangeant 8 volumes de borax (tétraborate de sodium $Na_2 B_4 O_7$, $10H_2 O$) 0,05 M à 2 volumes de borate 0,2 M.

La concentration optimale des molécules épuratrices dans cette solution dépend beaucoup de la nature de la molécule utilisée.

Le tableau suivant donne, à titre d'exemple, la concentration du bain de greffage assurant le plateau de l'isotherme de LANGMUIR pour différentes molécules épuratrices.

| Molécule épuratrice | Concentration du bain de greffage assurant le plateau de l'isotherme de LANGMUIR |
|---|---|
| Ig G | 100 à 200 mg/l suivant l'origine de l'Ig G. |
| Albumine bovine | 200 à 500 mg/l |
| ADN de 0,3 kB | 20 à 30 g/l |
| Glycoprotéine AB | 1 à 5 g/l |
| Protéine A | 200 à 500 mg/l |
| Haptènes synthétiques A et B | Concentration saturante |

Après fixation de la molécule épuratrice, on procède à un lavage du support d'épuration afin d'éliminer toutes les molécules dont la fixation ne serait pas une fixation de nature covalente.

Ainsi, un lavage au KCl 3M pendant 10 minutes permet de décrocher les molécules fixées par liaison ionique, de même, un lavage au KSCN 0,5 M pendant 10 minutes permet de désorber les molécules fixées par liaison hydrophobe.
Ces opérations de lavage permettent de ne conserver que les molécules fixées au support par une liaison chimique de nature covalente, qui est une liaison très solide. Ainsi, lors du traitement du liquide à épurer, on sera certain que les molécules épuratrices ne seront pas emportées par le flux de liquide à épurer mais qu'elles seront bien fixées de manière à pouvoir réagir avec les molécules à éliminer. Il est naturellement intéressant que la molécule fixée soit orientée de façon à permettre une grande disponibilité de son site de reconnaissance et de fixation des molécules à épurer.

Ainsi, lorsque la molécule épuratrice est un anticorps destiné à épurer l'antigène correspondant, cet anticorps est de préférence choisi parmi des immunoglobulines G ; grâce à la nature hydrophobe du support, dûe par exemple aux groupements acrylonitriles, le greffage de l'Ig G a lieu par son extrémité hydrophobe, c'est-à-dire par le fragment Fc, ce qui permet de garder les deux fragments Fab disponibles, pour la réaction avec les molécules à épurer. Cette orientation avantageuse des molécules épuratrices greffées est un élément déterminant de l'efficacité de l'épuration. Il est en effet, primordial que la molécule épuratrice soit disponible dans l'espace pour réagir avec les molécules à épurer.

Cette possibilité de liaison covalente entre le support et les molécules épuratrices à groupement aminé grâce à la présence des acyl-azotures, évite la nécessité d'utiliser un intermédiaire chimique, tel que le glutaraldéhyde, pour lier chimiquement la molécule épuratrice au support. Cet intermédiaire chimique peut cependant être utilisé dans le cas de molécules épuratrices de petite taille qui doivent être éloignées du support, afin d'être disponibles pour réagir avec les molécules à épurer.

La méthode de fixation des molécules épuratrices selon la présente invention a permis de fixer avec succès de nombreuses molécules spécifiques des molécules à épurer.
Le tableau suivant dresse une liste des molécules épuratrices que l'on peut envisager de greffer, en correspondance avec les molécules à épurer.

| Molécules épuratrices fixées | Molécules à épurer |
|---|---|
| Ig lapins anti Ig G humaines | Ig G humaines |
| Ig moutons anti Ig G humaines | Ig G humaines |
| Ig G lapins anti-albumine humaine | Albumine humaine |
| ADN bicaténaire pourvu d'une extrémité monocaténaire | anti-ADN natifs chez les lupiques |
| Glycoprotéines représentant les substances de groupes A et B | Ig G et Ig M anti-A et anti-B |
| Haptène A | Ig G et Ig M anti A |
| Haptène B | Ig G et Ig M anti-B |
| Protéine A | Ig G (3 classes sur 4) |

Les quantités de molécules épuratrices greffées sur le support selon la technique qui vient d'être décrite et après lavage au KCl et au KSCN sont par exemple les suivantes :

| | |
|---|---|
| anti-Ig G ou Ig G | 15 à 20 μg/cm2 |
| Protéine A | 20 à 30 μg/cm2 |
| ADN de 0,3 Kilobases | 60 à 70 μg/cm2 |

**EPURATION**

Les supports d'immuno-épuration sélective ainsi greffés de molécules épuratrices peuvent être introduits dans des dispositifs permettant par exemple de traiter le plasma sanguin obtenu après filtration du sang dans un dispositif classique de plasmaphérèse. On a ainsi réalisé des essais en introduisant dans des modules métalliques un empilement de supports d'épuration microporeux, sous forme de membranes planes, en l'occurrence un empilement de 30 membranes ayant chacune une surface apparente de 172 cm2. Grâce à ce type de module, il a été possible de traiter 750 ml de plasma en le faisant traverser un empilement de membranes avec un débit de 12,5 ml/minute.

Ce traitement a permis d'éliminer du plasma de patients atteints de Lupus Erythémateux, les immunoglobulines G anti-ADN natifs. De la même façon, il a été possible d'éliminer différentes immunoglobulines telles que des Ig G et Ig M dirigées contre les substances de groupes A et B.

Le tableau suivant regroupe les résultats des quantités de molécules épurées, ainsi que les quantités de molécules épuratrices greffées :

| Quantité de molécules épuratrices greffées | Quantités de molécules épurées |
|---|---|
| Anti Ig G : 15 à 20 μg/cm² ou Ig G | Ig G : 5 à 10 μg/cm² |
| Protéine A : 20 à 30 μg/cm² | Ig G : 30 μg/cm² |
| ADN de 0,3 kB 60 à 70 μg/cm² | Ig G anti ADN natifs baisse du titre de 80 à 100% |
| Glycoprotéine extraite d'estomac de ruminant (représentant les substances de groupes sanguins A et B) | Ig G et Ig M anti A et B baisse du titre quasi totale |

Bien que la présente invention soit susceptible de nombreuses modifications, les exemples qui suivent illustrent de façon non limitative, quelques modes de réalisation de l'invention.

Exemple 1

Méthode de préparation de membranes activées

Des disques de 60 mm de membranes microporeuses TPC 19 sont empilés dans une cellule inox, jusqu'à concurrence de 15 par exemple. Ces membranes sont tout d'abord dégazées au dioxyde de carbone $CO_2$ puis sont mouillées à l'éthanol absolu en faisant circuler dans le module pendant 15 minutes en circuit fermé de l'éthanol avec un débit de 4,7 ml/mn (78,33.$10^{-9}$ m³/s).
Puis les membranes sont rincées avec de l'eau pour préparation injectable pendant 15 minutes en circuit ouvert, le débit de circulation de l'eau à travers les membranes étant de 4,7 ml/mn (78,33.$10^{-9}$ m³/s).
On effectue le traitement à l'hydrazine en faisant passer 100 ml d'hydrazine à 2% en solution aqueuse pendant 2 heures en circuit fermé avec un débit de 4,7 ml/mn (78,33.$10^{-9}$ m³/s).
Les membranes sont ensuite rincées à l'eau pour préparation injectable circulant pendant 5 mn en circuit ouvert avec un débit de 4,7 ml/mn (78,33.$10^{-9}$ m³/s).
Les opérations suivantes sont ensuite effectuées à 4°C. On fait passer de l'acide nitreux 0,5 N 4mn en circuit fermé avec un débit de 4,7 ml/mn (78,33.$10^{-9}$ m³/s).
Puis on fait passer du tampon borate avec un débit de 2,7 ml/mn (45.$10^{-9}$ m³/s) pendant une durée pouvant par exemple être de 15 à 30 mn en circuit fermé jusqu'à l'obtention d'un pH égal à 9.
On obtient ainsi des membranes activées susceptibles de réagir avec les molécules épuratrices à greffer.

Exemple 2

Greffage de fragments d'ADN pourvus d'une extrémité aminée.

L'opération de greffage est effectuée entre 0° et 4°C sur des membranes obtenues selon l'exemple 1.

On fait passer 38 ml d'une solution d'ADN de 0,3 Kilobases à 30 g/l en tampon borate à travers les membranes ; cette circulation est effectuée pendant deux heures en circuit fermi avec un débit de 1,7 ml/mn ($28,33.10^{-9}$ $m^3/s$).
On obtient ainsi des membranes greffées de fragments d'ADN destinées à épurer des anticorps anti-ADN.

Exemple 3

Greffage de glycoprotéines représentant les substances de groupes sanguins A et B.

L'opération de greffage est effectuée entre 0 et 4° C sur des membranes obtenues selon l'exemple 1. On fait passer 38 ml d'une solution de glycoprotéines fournie par la Société Benasil International en tampon borate à travers les membranes ; cette circulation est effectuée pendant deux heures en circuit fermé avec un débit de 1,7 ml/mn ($28,33.10^{-9}$ $m^3/s$).
On obtient ainsi des membranes greffées de molécules épuratrices destinées à épurer les Ig G et Ig M anti-A et anti-B.

Exemple 4

Lavage des membranes greffées par des fragments d'ADN natifs.

Les membranes greffées selon le mode décrit à l'exemple 2 subissent différentes opérations de lavage qui sont effectuées à une température comprise entre 0 et 4°C.
On fait tout d'abord passer 100 ml d'eau physiologique c'est-à-dire une solution aqueuse de chlorure de sodium à 0,9% à travers les membranes, pendant 20 mn en circuit fermé avec un débit de 4,7 ml/mn ($78,33.10^{-9}$ $m^3/s$), puis à nouveau 100 ml d'eau physiologique.
On fait ensuite passer 100 ml d'une solution de KCl 3 M dans les mêmes conditions de circulation puis une solution de KSCN 0,5 M également dans les mêmes conditions.
Les membranes sont ensuite lavées grâce à une circulation fermée de 100 ml d'eau stérile pour préparation injectable pendant 20 mn avec un débit de 4,7 ml/mn ($78,33.10^{-9}$ $m^3/s$), puis à nouveau 100 ml d'eau physiologique.
Dans une seconde phase de rinçage, on fait circuler 2 l d'eau stérile pour préparation injectable en circuit ouvert à travers les membranes avec un débit de 4,7 ml/mn ($78,33.10^{-9}$ $m^3/s$).
Les membranes greffées et rincées sont ensuite lyophilisées puis stérilisées au rayonnement gamma jusqu'à obtention d'une dose de rayonnement de 30 kGray (J/kg).
Les membranes ainsi obtenues sont prêtes à l'utilisation pour l'épuration d'un liquide.

Exemple 5

Lavage des membranes greffées par des glycoprotéines A et B.

Les membranes greffées selon le mode décrit à l'exemple 3 sont lavées à froid, entre 0° et 4° C. On fait tout d'abord passer à deux reprises 100 ml d'eau physiologique à travers les membranes pendant 20 mn en circuit fermé, avec un débit de 4,7 ml/mn ($78,33.10^{-9}$ $m^3/s$). On fait ensuite passer 100 ml d'une solution de KCl 3M dans les mêmes conditions de circulation puis une solution de KSCN 0,5 M également dans les mêmes conditions.
Les membranes sont ensuite rincées grâce à une solution de sérum physiologique stérile. La cellule contenant les membranes est maintenue remplie de sérum physiologique jusqu'à son utilisation.

r Exemple 6

Méthode de préparation de membranes activées représentant une surface de 0, 516 $m^2$.

Des disques de membranes TPC 19 de diamètre 17cm sont empilés au nombre de 30 dans une cellule en acier inox.
Le mouillage des membranes est effectue en faisant circuler de l'éthanol absolu en circuit fermé à travers les membranes ; cette circulation se fait tout d'abord à débit faible puis ensuite à débit plus élevé, c'est-à-dire à environ 100 ml/mn ($1,66.10^{-6}$ $m^3/s$). La circulation de l'éthanol est maintenue jusqu'à ce que tout l'air soit chassé de la cellule. Les membranes sont ensuite rincées avec un litre d'eau distillée circulant à un débit de 20 ml/mn

($0,33.10^{-6}$ $m^3/s$) en circuit fermé, puis avec un litre d'eau distillée, circulant à 65 ml/mn en circuit ouvert.
L'hydrazinolyse du support est effectuée en faisant circuler deux litres d'une solution aqueuse d'hydrazine à 2% à travers les membranes, pendant deux heures, en circuit fermé.
Le débit de circulation est de 10 ml/mn ($0,167.10^{-6}$ $m^3/s$) pendant les 15 premières minutes, puis de 20 ml/mn ($0,333.10^{-6}$ $m^3/s$).
Les membranes sont ensuite rincées en faisant circuler un litre d'eau à 25 ml/mn ($0,417.10^{-6}$ $m^3/s$) pendant 10 minutes en circuit fermé puis un litre d'eau en circuit ouvert à 70 ml/mn ($1,167.10^{-6}$ $m^3/s$).
Les membranes hydrazidées sont ensuite traitées par de l'acide nitreux 0,5 M obtenu extemporanément par mélange de 600 ml de HCl 0,3 N et de 600 ml nitrite de sodium NaNO2 (3,4 g/100 ml d'eau stérile).
L'acide nitreux circule pendant 10 mn à travers les membranes en circuit fermé, avec un débit de 30 ml/mn ($0,5.10^{-6}$ $m^3/s$). Cette réaction, ainsi que les réactions suivantes est effectuée dans de la glace, c'est-à-dire à une température comprise entre 0 et 4°C.
Les membranes sont ensuite rincées par un litre d'eau distillée circulant en circuit ouvert à un débit pouvant atteindre 100 ml/mn ($1,667.10^{-6}$ $m^3/s$), puis avec 1,3 l de tampon borate 0,2 M circulant à environ 85 ml/mn ($1,417.10^{-6}$ $m^3/s$) en circuit fermé.
On contrôle que le pH est bien égal à 9.
On obtient ainsi des membranes activées susceptibles d'être greffées de molécules épuratrices.

Exemple 7

Greffage d'ADN de 0,3 Kilobases.

Le greffage est effectué sur des membranes obtenues selon l'exemple 6, à une température comprise entre 0 et 4°C.
On fait circuler 300 ml d'une solution à 30 g/l d'ADN de 0,3 Kilobases en tampon borate, à un débit de 5 à 10 ml/mn (83,3 à $166,6.10^{-9}$ $m^3/s$), en circuit fermé pendant deux heures.
On obtient alors des membranes greffées de fragments d'ADN.

Exemple 8

Greffage de glycoprotéines représentant les substances de groupes A et B.

Le greffage est effectué à une température comprise entre 0 et 4°C, sur les membranes préparées selon la méthode décrite à l'exemple 6.
On fait passer à travers les membranes 300 ml d'une solution de glycoproteine AB commercialisée par la Société BENASIL INTERNATIONAL mais concentrée 4 fois, pendant deux heures en circuit fermé à un débit compris entre 5 et 10 ml/mn (soit $83,3.10^{-9}$ et $166,6.10^{-9}$ $m^3/s$).
On obtient ainsi des membranes greffées de molécules épuratrices destinées à épurer les Ig G et Ig M anti-A et anti-B.

Exemple 9

Lavage des membranes greffées de fragments d'ADN natifs.

Les membranes greffées selon la méthode décrite à l'exemple 7 sont lavées à une température de 0 à 4°C.
Le lavage est effectue par passage en circuit ouvert d'un premier litre d'eau physiologique à un débit de 20 à 40 ml/mn (soit 0,33 à $0,67.10^{-6}$ $m^3/s$), puis d'un second litre dans les mêmes conditions.
On fait ensuite passer un litre de solution KCl 3M en circuit ouvert à travers les membranes puis un litre de solution KSCN 0,5M dans les mêmes conditions. Le débit de circulation est alors de 80 ml/mn ($1,33.10^{-6}$ $m^3/s$).
Le rinçage final est effectué par passage à travers les membranes d'un litre d'eau stérile, circulant à 80 ml/mn ($1,33.10^{-6}$ $m^3/s$).
On lyophilise ensuite l'ensemble des membranes avant de les stériliser au rayonnement gamma jusqu'à obtention d'une dose de rayonnement de 30 KGray. Les membranes peuvent ensuite être conservées stériles jusqu'à leur utilisation.

Exemple 10

Lavage des membranes greffées de glycoprotéine représentant les substances de groupe A et B.

Les membranes greffées selon la méthode décrite à l'exemple 8 sont lavées à une température comprise entre 0 et 4°C.
Le lavage est effectué par passage en circuit ouvert d'un premier litre d'eau physiologique à un débit de 20 à 40 ml/mn (soit 0,33 à $0{,}67.10^{-6}$ $m^3/s$), puis d'un second litre dans les mêmes conditions.
On fait ensuite passer un litre de solution KCl 3M en circuit ouvert à travers les membranes puis un litre de solution KSCN 0,5M dans les mêmes conditions.
Le rinçage final est effectué par passage à travers les membranes d'un litre de liquide physiologique stérile.
Le liquide physiologique utilisé peut être une solution de chlorure de sodium à 0,9 pourcent, ou par exemple un tampon phosphate disodique monopotassique obtenu par mélange de $Na_2$ $HPO_4$, $2H_2O$ (3,65 g pour 400 ml) et de $KH_2$ $PO_4$ (0,68 g pour 100 ml).
La cellule inox contenant les membranes est maintenue remplie de sérum physiologique jusqu'à son utilisation.

Exemple 11

Epuration en circuit ouvert d'un plasma provenant d'un patient atteint de Lupus Erythémateux Disséminé.

On extrait, grâce à la technique de plasmaphérèse, le plasma d'un lupique, qui est remplacé dans le système vasculaire du patient par une solution d'albumines.
Le plasma obtenu est traité par des membranes obtenues selon l'exemple 9, et disposées à l'intérieur de cellules inox représentant chacune 0,5 $m^2$ de surface totale. Ces cellules, au nombre de 4 sont disposées en parallèle sur le circuit de circulation du plasma, et représentent donc une surface totale de traitement de 2 $m^2$.
Le débit de circulation du plasma est de 30 ml/mn (soit $0{,}5.10^{-6}$ $m^3/s$).
Le plasma obtenu après épuration est recueilli dans des poches afin d'être éliminé.
Des prélèvements de plasma sont effectués en amont et en aval des cellules afin de doser la quantité d'anticorps anti-ADN présents dans le plasma avant et après le dispositif d'épuration.
Le dosage des anticorps anti-ADN est effectué grâce à la technique ELISA, par une lecture de la Densité Optique à 492 nm.
La diminution de la différence de DO mesurée entre les prélèvements effectués en amont et en aval du dispositif d'épuration au cours du temps est relatée dans le tableau suivant :

| Temps en mn d'épuration | D.O.en amont cellule d'épuration | D.O. en aval cellule |
|---|---|---|
| 0 | 1,3 | |
| 7 | 0,412 | 0,025 |
| 15 | 0,415 | 0,051 |
| 20 | 0,405 | 0,052 |
| 30 | 0,395 | 0,100 |
| 40 | 0,337 | 0,100 |
| 60 | 0,295 | 0,110 |
| 90 | 0,22 | 0,190 |

La diminution de la D.O. mesurée montre l'efficacité de l'épuration des anticorps anti-ADN, bien que le rapprochement entre les valeurs mesurées avant et après le dispositif d'épuration témoigne de la saturation progressive des supports d'épuration.

Exemple 12

Epuration en circuit fermé d'un plasma de lupique.

Cette épuration in vitro simule une épuration in-vivo. Les trois litres de plasma obtenu par plasmaphérèse à partir du sang d'un lupique sont mis dans un flacon à l'intérieur duquel le plasma est maintenu sous agitation. Le plasma est mis en circulation avec un débit de 30 ml/mn (0,5.$10^{-6}$ $m^3/s$)pour être épuré par passage à travers un dispositif d'épuration constitué par quatre cellules inox disposées en parallèle et contenant des membranes obtenues selon la méthode décrite à l'exemple 9.
Des prélèvements de plasma sont effectues en amont et en aval du dispositif d'épuration afin d'effectuer le dosage des anticorps anti-ADN présents dans le plasma. Ce dosage est effectué par la méthode ELISA, grâce à une mesure au spectrophotomètre de la Densité Optique à la longeur d'onde de 492 nm.
L'évolution de la concentration en anticorps anti-ADN dans le plasma au cours du temps, est relatée dans le tableau suivant reproduisant les valeurs en fonction du temps du pourcentage de D.O. par rapport à la D.O. mesurée sur un échantillon de plasma non traité, à 492 nm, avant et après passage dans le dispositif d'épuration.

| Temps en mn | % de D.O. avant épuration | % de D.O. après épuration |
|---|---|---|
| 0 | 100 | |
| 10 | 66 | 3 |
| 20 | 33 | 8 |
| 30 | 30 | 13 |
| 60 | 33 | 35 |
| 90 | 23 | 17 |
| 120 | 19 | 17 |
| 150 | 25 | 8 |
| 180 | 15 | 15 |

La baisse du titre, en l'occurence ici la D.O. de 85 pourcent montre une bonne épuration des anticorps anti-ADN.

## Revendications

1. Procédé de traitement d'un support pour l'immuno-épuration sélective d'un liquide, ledit support étant constitué par un copolymère d'au moins un monomère comprenant au moins une fonction choisie parmi les fonctions acide, amide, hydrazide ou ester, dans lesquelles la fonction carbonyle C = 0 est reliée à la chaîne polymérique par R pouvant être un alcoyle, un aryle ou un simple lien valenciel et d'un monomère à au moins un groupement apportant de l'hydrophobie, caractérisé en ce qu'il consiste à transformer l'une au moins desdites fonctions en groupements acyl-azotures.

2. Procédé selon la revendication 1, caractérisé en ce que ledit groupement apportant de l'hydrophobie est constitué par un groupement nitrile.

3. Procédé selon l'une des revendications précédentes, caractérisé en ce qu'il consiste à transformer l'une au moins desdites fonctions acide, amide ou ester en hydrazide grâce à l'action de l'hydrazine, puis à transformer ladite hydrazide en acyl-azoture grâce à l'action du nitrite de sodium en milieu acide.

4. Procédé selon la revendication 3, caractérisé en ce qu'il consiste en outre à greffer des molécules épuratrices par réaction avec lesdits groupements acyl-azotures.

5. Procédé selon la revendication 4, caractérisé en ce qu'il consiste à greffer lesdites molécules épuratrices par liaison covalente en faisant réagir lesdits groupements acyl-azotures du support avec des groupements aminés présents sur les molécules épuratrices.

6. Procédé selon l'une des revendications 4 ou 5, caractérisé en ce qu'il consiste à effectuer le greffage par mise en contact du support pour l'immuno-épuration sélective avec une solution de greffage constituée par des molécules épuratrices en solution.

7. Procédé selon l'une des revendications 4 à 6, caractérisé en ce qu'il consiste à utiliser comme molécules épuratrices des protéines, des fragments d'acide ribonucléique, des fragments d'acide désoxyribonucléique, des glycoprotéines ou des polysaccharides à fonction aminée.

8. Procédé selon l'une des revendications 4 à 7, caractérisé en ce qu'il consiste à utiliser comme molécules épuratrices des immunoglobulines G en solution dans une substance tampon, à une concentration comprise entre 100 et 200 mg/l.

9. Procédé selon l'une des revendications 4 à 7, caractérisé en ce qu'il consiste à utiliser comme molécules épuratrices des fragments d'acide désoxyribonucléique bicaténaires pourvus d'une extrémité aminée.

10. Procédé selon la revendication 9, caractérisé en ce que les fragments d'acide désoxyribonucléique sont des fragments de 0,3 kilobases en solution dans une substance tampon, à une concentration comprise entre 20 et 30 g/l.

11. Procédé selon l'une des revendications 4 à 7, caractérisé en ce qu'il consiste à utiliser comme molécules épuratrices des glycoprotéines représentant les substances de groupes sanguins A et B.

12. Procédé selon l'une des revendications 4 à 7, caractérisé en ce qu'il consiste à utiliser comme molécules épuratrices des haptènes synthétiques représentant les substances de groupes sanguins A et B.

13. Procédé selon l'une des revendications 4 à 12, caractérisé en ce qu'il consiste à utiliser une solution de greffage dont le pH est compris entre 8 et 11.

14. Support pour l'immuno-épuration sélective d'un liquide, constitué par un copolymère d'au moins un monomère comprenant au moins une fonction choisie parmi les fonctions acide, amide, hydrazide ou ester, dans laquelle la fonction carbonyle C = 0 est reliée à la chaine polymérique par R pouvant être un alcoyle, un aryle ou un simple lien valenciel et d'un monomère à au moins un groupement apportant de l'hydrophobie, caractérisé en ce que ladite fonction acide, amide, hydrazide ou ester est transformée en acylazoture.

15. Support selon la revendication 14, caractérisé en ce que ledit groupement apportant de l'hydrophobie est constitué par un groupement nitrile.

16. Support selon l'une des revendications 14 ou 15, caractérisé en ce que la fonction acide a été transformée en fonction ester.

17. Support selon l'une des revendications 14 à 16, caractérisé en ce que les fonctions amide ou ester ont été transformées en hydrazides par action de l'hydrazine.

18. Support selon l'une des revendications 14 à 17, caractérisé en ce qu'il est constitué par une structure microporeuse dont la taille des pores est comprise entre 500 et 10 000 Angströms.

19. Support selon l'une des revendications 14 à 18, caractérisé en ce qu'il a été greffé de molécules épuratrices susceptibles de reconnaître et de se lier spécifiquement avec des substances à éliminer présentes dans ledit liquide.

20. Support selon la revendication 19, caractérisé en ce que lesdites molécules épuratrices peuvent être des protéines, des fragments d'ADN, des fragments d'ARN, des glycoprotéines ou des polysaccharides à fonction aminée.

21. Support selon la revendication 19 ou 20, caractérisé en ce que lesdites molécules épuratrices sont des anticorps et lesdites substances à éliminer sont des antigènes.

22. Support selon l'une des revendications 19 à 21, caractérisé en ce que lesdites molécules épuratrices sont des immunoglobulines G fixées par liaison covalente par leur fragment hydrophobe Fc.

23. Support selon l'une des revendications 19 à 21, caractérisé en ce que lesdites molécules épuratrices sont des fragments d'ADN et lesdites substances à éliminer sont des anticorps anti-ADN.

24. Support selon l'une des revendications 14 à 23, caractérisé en ce qu'il comprend entre 0, 3 et 3 équivalents de groupements acide, amide, hydrazide ou ester par kg de polymère.

25. Support selon l'une des revendications 14 à 24, caractérisé en ce qu'il est constitué par un copolymère acrylonitrile-méthacrylate de methyle-methallyl sulfonate de sodium.

26. Support selon l'une des revendications 14 à 24, caractérisé en ce qu'il est constitué par un copolymère acrylonitrile-acrylate de méthyle.

27. Support selon l'une des revendications 14 à 24, caractérisé en ce qu'il est constitué par un copolymère acrylonitrile-méthacrylate de méthyle.

28. Utilisation du support selon l'une des revendications 14 à 27 pour l'immuno-épuration d'un liquide tel que du sang, du plasma sanguin, ou une solution contenant des molécules d'origine biologique que l'on veut séparer.

## Patentansprüche

1. Verfahren zur Behandlung eines Trägers zur selektiven Immunreinigung einer Flüssigkeit, wobei der Träger besteht aus einem Copolymer aus mindestens einem Monomer, das mindestens eine funktionelle Gruppe aufweist, die unter Carbonsäuregruppen, Amidgruppen, Hydrazidgruppen oder Estergruppen, bei denen die Carbonylfunktion C=0 durch R, das eine Alkylgruppe, eine Arylgruppe oder eine Einfachbindung sein kann, an die Polymerkette gebunden ist, ausgewählt ist, und einem Monomer mit mindestens einer Gruppe, die hydrophobe Eigenschaften verleiht,
**dadurch gekennzeichnet,**
daß es in der Umwandlung mindestens einer der genannten funktionellen Gruppen in Acylazidgruppen besteht.

2. Verfahren nach Anspruch 1, dadurch gekennzeichnet, daß die hydrophobe Eigenschaften verleihende Gruppe aus einer Nitrilgruppe besteht.

3. Verfahren nach Anspruch 1 oder 2, dadurch gekennzeichnet, daß es in der Umwandlung mindestens einer Funktion aus der Gruppe der Carbonsäuregruppen, der Amidgruppen oder der Estergruppen durch Einwirkung von Hydrazin in Hydrazidgruppen und anschließende Umwandlung der Hydrazidgruppen durch Einwirkung von Natriumnitrit in saurem Milieu in Acylazidgruppen besteht.

4. Verfahren nach Anspruch 3, dadurch gekennzeichnet, daß es ferner in der Pfropfung von Reinigungsmolekülen durch Umsetzung mit den Acylazidgruppen besteht.

5. Verfahren nach Anspruch 4, dadurch gekennzeichnet, daß es in der Pfropfung der Reinigungsmoleküle durch kovalente Bindung durch Umsetzung der Acylazidgruppen des Trägers mit an den Reinigungsmolekülen vorliegenden Aminogruppen besteht.

6. Verfahren nach Anspruch 4 oder 5, dadurch gekennzeichnet, daß es in der Pfropfung durch Inkontaktbringen des Trägers für die selektive Immunreinigung mit einer aus Reinigungsmolekülen in Lösung bestehenden Pfropflösung besteht.

7. Verfahren nach einem der Ansprüche 4 bis 6, dadurch gekennzeichnet, daß es in der Verwendung von Proteinen, Ribonucleinsäurefragmenten, Desoxyribonucleinsäurefragmenten, Glycoproteinen oder Polysacchariden mit Aminfunktionen als Reinigungsmoleküle besteht.

8. Verfahren nach einem der Ansprüche 4 bis 7, dadurch gekennzeichnet, daß es in der Verwendung von Immunglobulinen G als Reinigungsmoleküle in Lösung in einer Puffersubstanz bei einer Konzentration von 100 bis 200 mg/l besteht.

9. Verfahren nach einem der Ansprüche 4 bis 7, dadurch gekennzeichnet, daß es in der Verwendung von doppelsträngigen Fragmenten von Desoxyribonucleinsäure, die an einem Ende eine Aminogruppe aufweisen, als Reinigungsmoleküle besteht.

10. Verfahren nach Anspruch 9, dadurch gekennzeichnet, daß die Desoxyribonucleinsäurefragmente Fragmente von 0,3 Kilobasen sind, die in Lösung in einer Puffersubstanz in einer Konzentration von 20 bis 30 g/l vorliegen.

11. Verfahren nach einem der Ansprüche 4 bis 7, dadurch gekennzeichnet, daß es in der Verwendung von Glycoproteinen als Reinigungsmoleküle besteht, die Substanzen der Blutgruppen A und B darstellen.

12. Verfahren nach einem der Ansprüche 4 bis 7, dadurch gekennzeichnet, daß es in der Verwendung von synthetischen Haptenen als Reinigungsmoleküle besteht, die Substanzen der Blutgruppen A und B darstellen.

13. Verfahren nach einem der Ansprüche 4 bis 12, dadurch gekennzeichnet, daß es in der Verwendung einer Pfropflösung besteht, deren pH-Wert 8 bis 11 beträgt.

14. Träger zur selektiven Immunreinigung einer Flüssigkeit, der besteht aus einem Copolymer aus mindestens einem Monomer, daß mindestens eine funktionelle Gruppe aufweist, die unter Carbonsäuregruppen, Amidgruppen, Hydrazidgruppen oder Estergruppen, bei denen die Carbonylfunktion C=0 durch R, das eine Alkylgruppe, eine Arylgruppe oder eine Einfachbindung sein kann, an die Polymerkette gebunden ist, ausgewählt ist, und einem Monomer mit mindestens einer Gruppe, die hydrophobe Eigenschaften verleiht, dadurch gekennzeichnet, daß die Carbonsäuregruppen, Amidgruppen, Hydrazidgruppen oder Estergruppen in Acylazidgruppen umgewandelt sind.

15. Träger nach Anspruch 14, dadurch gekennzeichnet, daß die hydrophobe Eigenschaften verleihende Gruppe aus einer Nitrilgruppe besteht.

16. Träger nach Anspruch 14 oder 15, dadurch gekennzeichnet, daß die Carbonsäuregruppen in Estergruppen umgewandelt sind.

17. Träger nach einem der Ansprüche 14 bis 16, dadurch gekennzeichnet, daß die Amidgruppen oder Estergruppen durch Einwirkung von Hydrazin in Hydrazidgruppen umgewandelt sind.

18. Träger nach einem der Ansprüche 14 bis 17, dadurch gekennzeichnet, daß er aus einer mikroporösen Struktur besteht, deren Porengröße 500 bis 10000 Å beträgt.

19. Träger nach einem der Ansprüche 14 bis 18, dadurch gekennzeichnet, daß er mit Reinigungsmolekülen gepfropft ist, die befähigt sind, in der Flüssigkeit vorliegende, abzutrennende Substanzen zu erkennen und sie spezifisch zu binden.

20. Träger nach Anspruch 19, dadurch gekennzeichnet, daß die Reinigungsmoleküle Proteine, DNS-Fragmente, RNS-Fragmente, Glycoproteine oder Polysaccharide mit Aminfunktion sind.

21. Träger nach Anspruch 19 oder 20, dadurch gekennzeichnet, daß die Reinigungsmoleküle Antikörper und die abzutrennenden Substanzen Antigene sind.

22. Träger nach einem der Ansprüche 19 bis 21, dadurch gekennzeichnet, daß die Reinigungsmoleküle Immunglobuline G sind, die durch kovalente Bindung über ihr hydrophobes Fc-Fragment gebunden sind.

23. Träger nach einem der Ansprüche 19 bis 21, dadurch gekennzeichnet, daß die Reinigungsmoleküle DNS-Fragmente und die abzutrennenden Substanzen anti-DNS-Antikörper sind.

24. Träger nach einem der Ansprüche 14 bis 23, dadurch gekennzeichnet, daß er 0,3 bis 3 Äquivalent Säuregruppen, Amidgruppen, Hydrazidgruppen oder Estergruppen pro Kilogramm Polymer aufweist.

25. Träger nach einem der Ansprüche 14 bis 24, dadurch gekennzeichnet, daß er aus einem Acrylnitril-Methylmethacrylat-Natriummethallylsulfonat-Copolymer besteht.

26. Träger nach einem der Ansprüche 14 bis 24, dadurch gekennzeichnet, daß er aus einem Acrylnitril-Methylacrylat-Copolymer besteht.

27. Träger nach einem der Ansprüche 14 bis 24, dadurch gekennzeichnet, daß er aus einem Acrylnitril-Methylmethacrylat-Copolymer besteht.

28. Verwendung des Trägers nach einem der Ansprüche 14 bis 27 zur Immunreinigung einer Flüssigkeit wie Blut oder Blutplasma oder einer Lösung, die zu trennende Moleküle biologischen Ursprungs enthält.

## Claims

1. Process for treating a support for the selective immuno-purification of a liquid, said support being constituted by a copolymer of at least one monomer comprising at least one function selected from the acid, amide, hydrazide or ester functions, in which the carbonyl C = O function is attached to the polymeric chain by R which may be an alkyl, an aryl or a simple valence bond and of a monomer with at least one group contributing hydrophoby, characterized in that it consists in transforming at least one of said functions into acyl-nitride groups.

2. Process according to Claim 1, characterized in that said group contributing hydrophoby is constituted by a nitrile group.

3. Process according to one of the preceding Claims, characterized in that it consists in transforming at least one of said acid, amide or ester functions into hydrazide thanks to the action of hydrazine, then in transforming said hydrazide into acyl-nitride thanks to the action of sodium nitrite in acid medium.

4. Process according to Claim 3, characterized in that it further consists in grafting purifying molecules by reaction with said acyl-nitride groups.

5. Process according to Claim 4, characterized in that it consists in grafting said purifying molecules by covalent bond by reacting said acyl-nitride groups of the support with amino groups present on the purifying molecules.

6. Process according to one of Claims 4 or 5, characterized in that it consists in effecting grafting by placing the support for the selective immuno-purification in contact with a grafting solution constituted by purifying molecules in solution.

7. Process according to one of Claims 4 to 6, characterized in that it consists in using as purifying molecules proteins, fragments of ribonucleic acid, fragments of deoxyribonucleic acid, glycoproteins or polysaccharides with amino function.

8. Process according to one of Claims 4 to 7, characterized in that it consists in using as purifying molecules G-immunoglobulins in solution in a buffer substance, at a concentration of between 100 and 200 mg/l.

9. Process according to one of Claims 4 to 7, characterized in that it consists in using as purifying molecules bicatenary fragments of deoxyribonucleic acid provided with an amino end.

10. Process according to Claim 9, characterized in that the fragments of deoxyribonucleic acid are fragments

of 0.3 kilobases in solution in a buffer substance, at a concentration of between 20 and 30 g/l.

11. Process according to one of Claims 4 to 7, characterized in that it consists in using as purifying molecules glycoproteins representing the substances of blood groups A and B.

12. Process according to one of Claims 4 to 7, characterized in that it consists in using as purifying molecules synthetic haptenes representing the substances of blood groups A and B.

13. Process according to one of Claims 4 to 12, characterized in that it consists in using a grafting solution whose pH is included between 8 and 11.

14. Support for the selective immuno-purification of a liquid, constituted by a copolymer of at least one monomer comprising at least one function selected from the acid, amide, hydrazide or ester functions, in which the carbonyl C = O function is attached to the polymeric chain by R which may be an alkyl, an aryl or a simple valence bond and of a monomer with at least one group contributing hydrophoby, characterized in that said acid, amide, hydrazide or ester function is transformed into acyl-nitride.

15. Support according to Claim 14, characterized in that said group contributing hydrophoby is constituted by a nitrile group.

16. Support according to one of Claims 14 or 15, characterized in that the acid function has been transformed into ester function.

17. Support according to one of Claims 14 to 16, characterized in that the amide or ester functions have been transformed into hydrazides by action of hydrazine.

18. Support according to one of Claims 14 to 17, characterized in that it is constituted by a microporous structure of which the size of the pores is included between 500 and 10,000 Angströms.

19. Support according to one of Claims 14 to 18, characterized in that it was grafted with purifying molecules capable of recognizing and specifically bonding with substances to be eliminated present in said liquid.

20. Support according to Claim 19, characterized in that said purifying molecules may be proteins, DNA fragments, RNA fragments, glycoproteins or polysaccharides with amino function.

21. Support according to Claim 19 or 20, characterized in that said purifying molecules are antibodies and said substances to be eliminated are antigens.

22. Support according to one of Claims 19 to 21, characterized in that said purifying molecules are G immunoglobulins fixed by covalent bond by their hydrophobic Fc fragment.

23. Support according to one of Claims 19 to 21, characterized in that said purifying molecules are DNA fragments and said substances to be eliminated are anti-DNA antibodies.

24. Support according to one of Claims 14 to 23, characterized in that it comprises between 0, 3 and 3 equivalents of acid, amide, hydrazide or ester groups per kg of polymer.

25. Support according to one of Claims 14 to 24, characterized in that it is constituted by an acrylonitrile-methyl methacrylate-methallyl sodium sulfonate copolymer.

26. Support according to one of Claims 14 to 24, characterized in that it is constituted by an acrylonitrile-methyl acrylate copolymer.

27. Support according to one of Claims 14 to 24, characterized in that it is constituted by an acrylonitrile-methyl methacrylate copolymer.

28. Use of the support according to one of Claims 14 to 27 for the immuno-purification of a liquid such as blood, blood plasma, or a solution containing molecules of biological origin which it is desired to separate.